# FASCICULE DE BREVET EUROPEEN

(11) **EP 3 393 446 B1**
(45) Date de publication et mention de la délivrance du brevet: **22.02.2023**
(21) Numéro de dépôt: 16829084.9
(22) Date de dépôt: 23.12.2016
(51) Int. Cl.: A61K 8/49, A61Q 17/00, A61K 8/44, A61K 8/06

(54) **COMPOSITION A BASE D'ACIDE SPICULISPORIQUE**
SPICULISPORSÄUREBASIERTE ZUSAMMENSETZUNG
SPICULISPORIC ACID-BASED COMPOSITION

(30) Priorité: 23.12.2015 FR 1563240; 23.12.2015 FR 1563245
(43) Date de publication de la demande: 31.10.2018
(73) Titulaire: L'Oréal, 75008 Paris (FR)
(72) Inventeur: AGNAOU, Reda, 94152 Chevilly la Rue (FR); MARION, Catherine, 94152 Chevilly la Rue (FR)
(74) Mandataire: Nony
(86) Numéro de dépôt international: PCT/EP2016/082590
(87) Numéro de publication internationale: WO 2017/109192

(56) Documents cités:
- WO-A1-2015/067784
- WO-A1-2015/067785
- WO-A1-2016/005707
- JP-A- H0 559 389

## Description

La présente invention concerne le domaine des compositions filmogènes utilisables en cosmétique, plus particulièrement elle vise les émulsions présentant des propriétés filmogènes.

L'obtention de compositions susceptibles de former un film stable et homogène est un problème technique récurrent dans de nombreux domaines et plus particulièrement en cosmétique comme par exemple pour des applications en tant que crèmes, déodorants, mascaras, vernis à ongles, produits de soin et/ou de maquillage.

En effet de nombreux produits cosmétiques, grâce à cette propriété filmogène, vont voir leurs propriétés améliorées que ce soit en termes de dispersion des composants, de toucher ou encore d'effet barrière entre le support sur lequel a été appliquée la composition et le milieu extérieur.

Généralement pour obtenir ce genre de compositions, on utilise principalement des polymères filmogènes conventionnels, en outre quand l'obtention d'une émulsion est visée, on utilise de préférence des molécules tensio-actives synthétiques à propriété filmogène généralement issues du pétrole.

Cependant la plupart des molécules tensio-actives synthétiques à propriété filmogène issues du pétrole utilisées dans les diverses industries sont délétères pour l'environnement.

De plus, les tensioactifs synthétiques sont certes de bons émulsionnants mais conduisent pour la plupart à la formation de films sensibles à l'humidité et faiblement cohésifs.

Il demeure par conséquent un besoin de systèmes émulsionnants filmogènes, qui soient respectueux de l'environnement.

Par ailleurs, la peau et le cuir chevelu humain sont les premières barrières protégeant le corps de l'environnement. Chaque jour, ces derniers subissent des agressions externes, qui sont responsables de nombreux problèmes de peau, tels que le vieillissement accéléré, des affections de la peau, un incomfort, ou l'état graisseux de la peau, etc.

En particulier, la pollution atmosphérique, à savoir la quantité de polluants dans l'air, soulève des inquiétudes de plus en plus nombreuses pour les consommateurs, en raison de son impact négatif sur la peau. Parmi les différents types de polluants existant dans l'air, la poussière ou les fines particules telles que les particules ayant un diamètre inférieur à 10 µm (PM10), de préférence inférieur à 2,5µm (PM2.5), les poudres de carbone, les gaz tels que CO, SO₂, NOₓ, ont attiré l'attention des consommateurs, en particulier ces dernières années.

Les particules fines existant dans l'air tendent à adhérer à la peau. Elles se déposent ou restent déposées sur la peau même après nettoyage. Ce dépôt n'est pas désiré par le consommateur, dans la mesure où l'on pense que les pores de la peau peuvent être bouchés et conduire ainsi à des problèmes de peau. La pollution atmosphérique est également à l'origine de problèmes de vieillissement cutané (notamment lié au stress oxydatif généré par les particules polluantes, les UV, tabac...), de peau deshydratée ou encore d'augmentation de la séborrhée (Lefebvre et al., 2015, International Journal of Cosmetic science, 37 :329-338).

L'état de la technique ne divulgue pas de solutions permettant de prévenir ou de réduire le dépôt des particules fines sur la peau et le cuir chevelu.

Il existe donc un besoin fort pour la préparation de compositions possédant un effet anti-déposition contre les particules fines. Plus particulièrement, il existe un besoin pour des compositions présentant des propriétés bénéfiques d'anti-déposition en plus d'autres propriétés, telles que par exemple des propriétés d'hydratation ou d'amélioration de la douceur, après application sur les matières kératiniques, en particulier sur la peau ou le cuir chevelu. Il est en outre avantageux que de telles compositions soient capables de présenter les effets décrits ci-dessus pendant une durée relativement longue, par exemple pendant 6 à 8 heures.

Les compositions cosmétiques comprennent souvent des composants volatils tels que des solvants volatils dans la phase aqueuse des compositions de soin ou de maquillage, des huiles volatiles dans des compositions de maquillage ou encore pour certaines applications des huiles essentielles.

Toutefois leur volatilité peut poser un certain nombre de problèmes.

En effet, lorsque l'effet recherché n'est pas lié à la volatilité du composé, il est souvent souhaité de diminuer cette volatilité afin de s'affranchir de devoir introduire une trop grande quantité de matières volatiles dans les produits les comprenant, et notamment dans les compositions cosmétiques et/ou dermatologiques.

Concernant plus particulièrement les huiles essentielles, elles sont généralement odorantes mais pas toujours parfumantes : en effet elles n'engendrent pas toujours une odeur plaisante pour l'entourage. Elles sont utilisées dans une grande variété de produits alimentaires, désodorisants et cosmétiques. Leur composition complexe leur confère des propriétés biologiques spécifiques selon l'origine de la plante telle que des propriétés anti-âge, anti-bactérienne, déodorante...

Cependant les constituants de ces actifs végétaux sont essentiellement hydrophobes et présentent des volatilités plus ou moins élevées qui rendent généralement leur formulation difficile.

Ainsi, dans ce contexte, il serait souhaitable de diminuer la volatilité des matières volatiles odorantes. Cela permettrait avantageusement, d'une part, de conserver une plus grande quantité de ladite matière volatile au niveau de son site d'application et ainsi d'augmenter son efficacité, et d'autre part de diminuer le coût de revient des compositions les contenant.

Les auteurs de la présente invention ont montré que ces problèmes pouvaient être résolus en utilisant des sels particuliers d'acide spiculisporique.

L'acide spiculisporique est un biotensioactif émulsionnant issu de microorganisme qui est non toxique pour l'environnement.

Par conséquent ce biotensioactif apparaît comme une alternative intéressante aux molécules tensio-actives synthétiques à propriété filmogène issues du pétrole dans la mesure où ces dernières sont généralement délétères pour l'environnement.

La demande WO2016005707 décrit l'utilisation cosmétique de l'acide spiculisporique comme actif déodorant, et en particulier décrit l'utilisation cosmétique comme actif déodorant de l'acide spiculisporique, l'acide spiculisporique étant sous forme libre, partiellement neutralisé ou totalement neutralisé par au moins une base minérale et/ou une base organique, et plus particulièrement dans une composition comprenant un milieu physiologiquement acceptable.

De plus les tensioactifs synthétiques sont certes de bons émulsionnants mais conduisent pour la plupart à la formation de films sensibles à l'humidité et faiblement cohésifs.

Ainsi la présente invention concerne une composition en particulier cosmétique, sous la forme d'une émulsion huile dans l'eau comprenant au moins 1% en poids d'une phase huileuse par rapport au poids total de la composition, et une phase aqueuse caractérisée en ce qu'elle comprend :
de 0,1 à 15 %, de préférence de 0,5 à 10 % et de manière encore plus préférée de 1 à 8 % en poids d'acide spiculisporique par rapport au poids total de la composition,
au moins une base choisie parmi les acides aminés et les alcanolamines en une quantité apte à former au moins le monosel de l'acide spiculisporique, ladite base étant en particulier présente en une quantité allant de 0,1 à 20 %, de préférence de 0,5 à 15 et de manière encore plus préférée de 0,6 à 10 % en poids par rapport au poids total de la composition,
moins de 3 %, de préférence moins de 1 % en poids de glycol(s) et/ou de polyol(s) par rapport au poids total de la composition, et étant avantageusement dénuée de glycols et/ou de polyol(s),
au moins un filtre UV et optionellement un additif choisi parmi les matières volatiles odorantes telles que les huiles essentielles et les substances parfumantes, les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

Le présent texte décrit par ailleurs une composition en particulier cosmétique, sous la forme d'une émulsion huile dans l'eau comprenant une phase huileuse, une phase aqueuse caractérisée en ce qu'elle comprend :
de 0,1 à 15 %, de préférence de 0,5 à 10 % et de manière encore plus préférée de 0,7 à 8 % en poids d'acide spiculisporique par rapport au poids total de la composition,
au moins une base choisie parmi les acides aminés et les alcanolamines en une quantité apte à former au moins le monosel de l'acide spiculisporique,
ladite composition comprenant moins de 3 %, de préférence moins de 1 % en poids de glycol(s) et/ou de polyol(s) par rapport au poids total de la composition, la composition étant avantageusement dénuée de glycols et/ou de polyols.

Le présent texte décrit également une composition en particulier cosmétique, sous la forme d'une émulsion huile dans l'eau comprenant au moins 1% d'une phase huileuse, et une phase aqueuse caractérisée en ce qu'elle comprend :
de 0,1 à 15 %, de préférence de 0,5 à 10 % et de manière encore plus préférée de 1 à 8 % en poids d'acide spiculisporique par rapport au poids total de la composition,
au moins une base choisie parmi les acides aminés et les alcanolamines en une quantité apte à former au moins le monosel de l'acide spiculisporique,
moins de 3 %, de préférence moins de 1 % en poids de glycol(s) et/ou de polyol(s) par rapport au poids total de la composition, et étant avantageusement dénuée de glycols et/ou de polyol(s),
au moins un filtre UV et/ou un additif choisi parmi les matières volatiles odorantes telles que les huiles essentielles et les substances parfumantes, les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

La composition selon l'invention est apte à former un film stable, homogène et résistant sur les matières kératiniques. Les compositions de l'invention possèdent ainsi des propriétés anti-déposition augmentées contre les particules fines, en particulier par la formation de films anti-adhésifs sur les matières kératiniques permettant d'éviter le dépôt des composés polluants sur lesdites matières kératiniques.

Les composants utilisés présentent notamment des propriétés filmogènes de la phase grasse et permetttent de stabiliser un film que l'on peut qualifier de « film gras ».

Dans un mode de réalisation particulier, les compositions selon l'invention comprennent également de 0,0001 % à 30 % d'au moins une matière volatile odorante choisie parmi les huiles essentielles et les substances parfumantes, de préférence les huiles essentielles, par rapport au poids total de la composition.

La composition conforme à l'invention permet avantageusement de diminuer la volatilité d'une matière volatile odorante en particulier d'une huile essentielle tout en réduisant son altération.

Les compositions selon l'invention permettent ainsi de limiter voire d'éviter l'évaporation de ladite matière volatile odorante.

La présente invention vise ainsi à fournir des compositions destinées à une utilisation pour le soin et/ou le maquillage des matières kératiniques, présentant des propriétés de résistance améliorée à la poussière et aux particules fines, c'est-à-dire possédant un effet anti-dépôt des particules fines sur les matières kératiniques.

Par conséquent, l'utilisation de l'acide spiculisporique dans le cadre de l'invention permet également de s'affranchir de l'ajout de polymères filmogènes supplémentaires. En effet il est toujours avantageux de pouvoir formuler des compositions comprenant un nombre limité d'ingrédients.

De plus les propriétés émulsionnantes de ce biotensioactif sont au moins de l'ordre de celles des tensioactifs synthétiques, les émulsions H/E selon l'invention sont par conséquent particulièrement stables et homogènes comme montré dans les exemples ci-après.

La composition selon l'invention constitue une alternative intéressante aux compositions de l'art antérieur comprenant des tensioactifs synthétiques de par son pouvoir émulsionnant, et de par la stabilité, la rémanence et la résistance à l'humidité des films obtenus après séchage de ladite composition.

La composition selon l'invention est en outre particulièrement respectueuse de l'environnement.

La composition selon l'invention présente en outre l'avantage de pouvoir être formulée en intégrant les ingrédients adéquats pour des applications cosmétiques très diverses telles que des déodorants, mascaras, vernis à ongles, des produits de soin et/ou de maquillage comme des crèmes, des fonds de teint ou des produits de soin capillaire, et de manière particulière les produits contenant des matières volatiles odorantes tels que des parfums ou avantageusement des huiles essentielles.

Les compositions obtenues, notamment les produits de soin et/ou de maquillage conduisent en outre à des films présentant des avantages en termes de propriétés sensorielles, notamment un toucher agréable.

Le film obtenu en appliquant les compositions de soin et/ou de maquillage présente une rupture rapide (propriété connue sous le nom de « quick breaking » en langue anglaise) mais ce film constitue néanmoins un frein à l'étalement. Cette propriété de constituer un frein à l'application conduit le consommateur à appliquer ladite composition pendant une durée plus longue et avec une pression sur la peau plus importante, ce qui permet une meilleure pénétration des actifs. La disparition du frein peut également constituer un signe d'application achevée et réussie.

Selon un deuxième aspect, la présente invention vise l'utilisation de la composition selon l'invention en tant que produit de maquillage, de soin, d'hygiène et/ou de nettoyage des matières kératiniques notament de la peau ou des cheveux, en particulier en tant que produit de nettoyage des matières kératiniques.

Selon un troisième aspect, la présente invention vise un procédé cosmétique non thérapeutique, de maquillage, de soin, d'hygiène et/ou de nettoyage comprenant une étape d'application de la composition selon l'invention sur lesdites matières kératiniques.

Plus particulièrement la composition selon l'invention est une composition de protection des matières kératiniques, plus particulièrement de la peau, vis-à-vis des polluants de l'air.

Ainsi la présente invention vise encore l'utilisation non-thérapeutique de la composition selon l'invention pour protéger les matières kératiniques vis-à-vis des composés polluants atmosphériques notamment choisis parmi le noir de carbone, les oxydes de carbone, les oxydes d'azote, les oxydes de soufre, les composés hydrocarbonés, les composés organiques volatiles, les métaux lourds, les particules fines PM2.5, PM10 et leurs mélanges.

Plus particulièrement ladite utilisation vise à protéger les matières kératiniques des désordres dûs aux composés polluants atmosphériques, lesdits désordres étant choisis parmi les peaux grasses, la déshydratation de la peau, l'altération de la désquamation, la diminution du squalène, la diminution de la vitamine E, la pigmentaion, l'augmentation de l'acide lactique.

La présente invention vise aussi un procédé cosmétique non thérapeutique de protection des matières kératiniques vis-à-vis des composés polluants atmosphériques notamment choisis parmi le noir de carbone, les oxydes de carbone, les oxydes d'azote, les oxydes de soufre, les composés hydrocarbonés, les composés organiques volatiles, les métaux lourds, les particules fines PM2.5, PM10 et leurs mélanges comprenant une étape d'application de la composition selon l'invention sur lesdites matières kératiniques.

Plus particulièrement ledit procédé vise à protéger les matières kératiniques des désordres dûs aux composés polluants atmosphériques, lesdits désordres étant choisis parmi les peaux grasses, la déshydratation de la peau, l'altération de la désquamation, la diminution du squalène, la diminution de la vitamine E, la pigmentation, l'augmentation de l'acide lactique.

La présente invention vise encore l'utilisation de l'acide spiculisporique sous forme partiellement ou totalement neutralisée par une base choisie parmi les acides aminés et les alcanolamines dans une composition, en particulier cosmétique, comme agent filmogène pour former un film sur la surface des matières kératiniques, notament sur la peau ou les cheveux, plus particulièrement cette utilisation est telle que le film formé protège les matières kératiniques vis-à-vis des composés polluants atmosphériques ou telle que le film formé favorise la répartition homogène d'au moins un actif compris dans la composition sur les matières kératiniques, ledit actif étant préférentiellement un filtre UV, de préférence lipophile, et/ou un additif choisi parmi les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

Il est également décrit dans le présent texte l'utilisation de l'acide spiculisporique sous forme partiellement ou totalement neutralisé par une base choisie parmi les acides aminés et les alcanolamines pour prévenir et/ou limiter et/ou éviter l'évaporation d'une matière volatile odorante contenue dans une composition cosmétique.

Le présent texte décrit par ailleurs l'utilisation de l'acide spiculisporique sous forme d'un mono-sel d'une base choisie parmi les acides aminés et les alcanolamines pour prévenir et/ou limiter et/ou éviter l'évaporation d'une matière volatile odorante contenue dans une composition cosmétique.

D'autres variantes, avantages et propriétés de l'invention seront mis en évidence dans la description et les exemples qui suivent.

### Acide spiculisporique

La composition sous forme d'émulsion selon l'invention comprend de l'acide spiculisporique, également connu sous le nom de 4,5-dicarboxy-4-pentadécanolide, de formule :

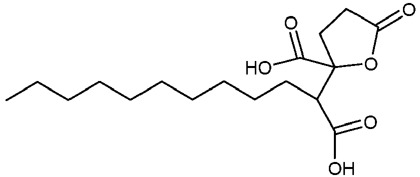

Il est connu que l'acide spiculisporique, encore appelé « acide Sp » dans la suite du texte, est insoluble, à température ambiante, dans l'eau et les corps gras mais qu'il est soluble dans l'éthanol.

En outre, il est connu que l'acide spiculisporique peut être solubilisé, à température ambiante, par salification.

Plus exactement, l'acide Sp peut former un mono-sel, un di-sel ou un tri-sel.
Le mono-sel correspond au produit de neutralisation du groupement carboxylique lié à l'atome de carbone en position C4 de l'acide Sp ;
le di-sel correspond au produit de neutralisation des groupements carboxyliques liés aux atomes de carbone en position C4 et C5 de l'acide Sp ;
le tri-sel correspond au produit de neutralisation des groupements carboxyliques liés aux atomes de carbone en position C4 et C5 de l'acide Sp et de salification de la fonction lactone sous sa forme ouverte.

Dans les émulsions selon l'invention, l'acide spiculisporique peut être sous forme d'un mono-sel, d'un di-sel, d'un tri-sel ou d'un mélange de plusieurs sels de degré de salification différent.

Selon un mode de réalisation préféré de l'invention, l'acide spiculisporique est sous forme d'un mono-sel.

Comme déjà mentionné, la quantité d'acide spiculisporique va de 0,1 à 15 %, de préférence de 0,5 à 10 % et de manière encore plus préférée de 0,7 à 8 % en poids d'acide spiculisporique par rapport au poids total de la composition.

En particulier, la quantité d'acide spiculisporique est de 0,6 ;0,7 ;0,8 ;0,9 ;1,0 ;1,1 ;1,2 ;1,3 ;1,4 ;1,5, 2 ;3 ;4 ; 5 ; 6 ; 7 ; 8 ; 9 ; 10 % en poids par rapport au poids total de la composition.

### Base

L'émulsion selon la présente invention comprend au moins une base choisie parmi les acides aminés et les alcanolamines, ladite base étant présente en une quantité apte à former au moins le monosel de l'acide spiculisporique.

La base utilisée est une base neutralisante, c'est-à-dire qu'elle permet de neutraliser l'acide spiculisporique pour former un sel dudit acide.

La ou les base(s) est/sont ainsi utililisée(s) en une quantité apte à former le mono-sel, le di-sel, le tri-sel ou un mélange de plusieurs sels d'acide spiculisporique de degrés de salification différents.

Selon un mode de réalisation préféré de l'invention, la quantité de base est choisie de façon à former le monosel de l'acide spiculisporique.

Dans le cadre de l'invention la base est choisie parmi les acides aminés et les alcanolamines.

Plus particulièrement la base peut être choisie parmi les acides aminés basiques suivants l'arginine, la lysine, l'ornithine, la citruline et l'histidine. De manière préférée, l'acide aminé est l'arginine.

La base peut aussi être choisie parmi les alcanolamines suivantes : les mono-, di- et triéthanolamines, l'isopropanolamine, le 2-amino-2-méthyl-1-propanol, ainsi que leurs mélanges. De manière préférée, l'alcanolamine est la triéthanolamine.

Avantageusement la quantité de base va de 0,1 à 20 %, de préférence de 0,5 à 15 et de manière encore plus préférée de 0,6 à 10 % en poids par rapport au poids total de la composition.

### Milieu physiologiquement acceptable

Outre les composés indiqués précédemment, une composition selon l'invention comprend un milieu physiologiquement acceptable.

Par « milieu physiologiquement acceptable », on entend désigner un milieu convenant particulièrement à l'application d'une composition de l'invention sur la peau et/ou les lèvres, comme l'eau, les huiles ou les solvants organiques couramment employés dans les compositions cosmétiques.

Le milieu physiologiquement acceptable (tolérance, toxicologie et toucher acceptables) est généralement adapté à la nature du support sur lequel doit être appliquée la composition, ainsi qu'à l'aspect sous lequel la composition doit être conditionnée.

### Phase huileuse

La composition conforme à l'invention peut comprendre une phase huileuse, également appelée phase grasse.

Comme montré dans les exemples, la composition peut contenir jusqu'à 70%, de préférence jusqu'à 60 % en poids de phase grasse par rapport au poids total de la composition. Les compositions, même comprenant un taux de phase grasse élevés peuvent conduire à des films stables, homogènes et résistants.

La phase grasse de la composition selon l'invention comprend l'ensemble des composés liposolubles ou lipodispersibles présents dans la composition, et en particulier les corps gras liquides à température ambiante (25 °C) et pression atmosphérique ou huiles (qui forment la phase huileuse).

Les huiles présentes dans la composition conforme à l'invention peuvent être siliconées ou hydrocarbonées.

Par huile siliconée (ou huile de silicone), on entend une huile contenant au moins un atome de silicium, et notamment contenant des groupes Si-O.

Comme huiles de silicone, on peut citer par exemple les huiles de silicone volatiles comme les cyclopolydiméthylsiloxanes (nom INCI : cyclomethicone), telles que le cyclopentasiloxane, le cyclohexasiloxane, l'octaméthylcyclotétrasiloxane, le décaméthylcyclopentasiloxane, le dodéca-méthylcyclohexasiloxane ; les silicones linéaires telles que l'heptaméthylhexyl-trisiloxane, l'heptaméthyloctyl-trisiloxane, l'hexaméthyldisiloxane, l'octaméthyl-trisiloxane, le décaméthyltétrasiloxane, le dodécaméthyl pentasiloxane ; les huiles de silicone non volatiles comme les polyméthylsiloxanes (PDMS), et les polyméthylsiloxanes phénylés tels que les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ; les polysiloxanes modifiés par des acides gras, des alcools gras ou des polyoxyalkylènes, et leurs mélanges.

Par « volatile », on entend un composé susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10-3 à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Comme huiles utilisables dans la composition de l'invention, on peut citer par exemple :
- les huiles hydrocarbonées d'origine végétale, telles que le squalane, les triglycérides liquides d'acides gras comportant de 4 à 30 atomes de carbone comme les triglycérides des acides heptanoïque ou octanoïque ou encore, par exemple, les huiles de jojoba, de babassu, de tournesol, d'olive, de noix de coco, de noix du brésil, de marula, de maïs, de soja, de courge, de pépins de raisin, de lin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité ;
- les esters et les éthers de synthèse, notamment d'acides gras, comme les huiles de formules R1COOR2 et R1OR2 dans laquelle R1 représente le reste d'un acide gras ou d'un alcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;

- les hydrocarbures linéaires ou ramifiés, d'origine minérale ou synthétique, volatils ou non volatils, et leurs dérivés, comme les alcanes ramifiés comportant de 8 à 18 atomes de carbone, par exemple les iso-alcanes (appelés aussi isoparaffines) en C8-C18 comme l'isododécane, l'isodécane, l'isohexadécane, tels que les isoparaffines vendues sous les noms commerciaux Isopar par la société Exxon Chemical ou les huiles vendues sous les noms commerciaux Permethyl par la société Presperse, l'isohexadécane et l'isododécane commercialisés par la société INEOS ; ainsi que l'huile de vaseline et le polyisobutène hydrogéné tel que l'huile de Parléam^{®} ; les alcanes linéaires volatiles comprenant de 7 à 17 atomes de carbone comme l'undécane, le tridécane ;
- les alcools gras liquides à température ambiante ayant de 8 à 26 atomes de carbone, comme l'octyldodécanol, le 2-butyloctanol, le 2-hexyldécanol, le 2-undécylpentadécanol ou l'alcool oléique ; et
- leurs mélanges.

On peut citer en particulier les huiles suivantes :
- les esters issus de la réaction d'au moins un acide gras comportant au moins 6 atomes de carbone, de préférence de 6 à 26 atomes de carbone et mieux de 6 à 20 atomes de carbone, encore mieux de 6 à 16 atomes de carbone et d'au moins un alcool comprenant de 1 à 17 atomes de carbone et mieux de 3 à 15 atomes de carbone ; on peut citer notamment le myristate d'isopropyle, le palmitate d'isopropyle, le caprate/caprylate d'ethyl2-hexyle (ou caprate/caprylate d'octyle), le palmitate d'éthyl-2-hexyle, le néopentanoate d'isostéaryle, l'isononanoate d'isononyle, le laurate d'hexyle, les esters d'acide lactique et d'alcools gras comprenant 12 ou 13 atomes de carbone, le carbonate de dicaprylyle tel que celui qui est commercialisé sous la dénomination CETIOL CC par la société COGNIS,
- les éthers d'acide gras comprenant de 6 à 20 atomes de carbone tel que le dicaprylyl éther (Cetiol OE de Cognis),
- les éthers de glycérol comprenant de 6 à 12 atomes de carbone comme le 2-éthyl hexyle éther de glycérol (nom INCI : ethylhexylglycerin) tel que le Sensiva SC 50 de la société Schulke & Mayr GmbH ;
- l'octydodécanol,
- les alcanes tels que ceux qui sont décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, euxmêmes obtenus à partir d'huile de coprah ou de palme.

A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande WO2008/155059 de la Société Cognis ;
- les polyesters obtenus par condensation de dimère et/ou trimère d'acide gras insaturé et de diol comme par exemple les polyesters d'acide dilinoléique et de diol commercialisés par Biosynthis sous la dénomination Viscoplast et notamment le polymère portant le nom INCI dilinoleic acid/propanediol copolymer ; et
- leurs mélanges.

De préférence, l'huile est choisie parmi les huiles végétales telles que citées ci-dessus.

La quantité de phase grasse dans la composition de l'invention est d'au moins 1% en poids, de préférence d'au moins 5% en poids, et peut aller de 1 % à 70 % en poids, de préférence de 5 à 70%, et de manière préférée de 5 % à 60 % en poids par rapport au poids total de la composition.

Après étalement sur les matières kératiniques et séchage, la composition selon l'invention forme avantageusement un film, et de préférence un film hydrophile présentant une forte mouillabilité (et possédant donc une surface de forte énergie), c'est-à-dire présentant une forte affinité pour l'eau.

Par " mouillabilité ", au sens de l'invention, on entend l'aptitude d'une surface à être mouillée par une matière donnée, en particulier par l'eau. De façon générale, lorsqu'un liquide est mis en contact avec la surface d'un solide (ici, du film formé), il se forme un angle de raccordement du premier sur le second. Lorsque le mouillage est parfait, l'angle de raccordement devient nul. Dans ce cas, l'énergie d'adhésion est maximale. La mouillabilité est évaluée par des méthodes bien connues de l'homme du métier, consistant essentiellement en la mesure de l'angle de contact d'une goutte d'eau déposée sur la surface du film formé après étalement de la composition et séchage. Cet angle de contact, généralement dénommé angle alpha, correspond à l'angle existant entre la surface du film et la tangente à la goutte à l'interface surface/eau/air. Cet angle peut être compris entre 0 et 180°.

Si l'angle est nul, alors le mouillage est total, c'est-à-dire que le liquide s'étale complètement sur la surface et qu'il existe donc des interactions fortes entre le support et le liquide. Si l'angle est de 180° alors le mouillage est nul, c'est-à-dire que le liquide forme une bille. Il n'y a qu'un point de contact entre le liquide et le support et surtout aucune affinité.

Pour des angles intermédiaires, le mouillage est partiel. Ainsi, on considère que lorsque cet angle de contact est supérieur à 45°, alors le matériau a une surface de faible énergie. Inversement, lorsque cet angle de contact est inférieur à 45°, alors le matériau a une surface de forte énergie. De façon générale les matériaux de faible énergie possèdent un caractère hydrophobe. Par surface de nature "hydrophobe", on entend, au sens de l'invention, une surface caractérisée par un angle de contact d'une goutte d'eau supérieure ou égale à 45° et généralement supérieure à 70°. Le terme "hydrophile" est quant à lui employé pour désigner une surface caractérisée par un angle de contact d'une goutte d'eau inférieure à 45°, et de préférence inférieure ou égale à 30°.

Les protocoles permettant d'évaluer le mouillage du film formé par les compositions selon l'invention sont bien connus dans l'état de l'art. Un protocole d'évaluation du mouillage peut notamment se présenter comme suit.

La composition selon l'invention est appliquée sur la surface d'un support, de préférence plane, ledit support étant de préférence une matière kératinique ou tout autre support approprié, à une température comprise entre 15 °C et 50 °C (de préférence entre 19 et 25 °C), dans des conditions d'humidité comprises entre 10% et 70%, et de préférence entre 50% et 60% et dans des conditions de pression atmosphérique standard. Le dépôt de la composition sur la surface du support peut avantageusement être réalisé à l'aide d'un tire-film,

Le séchage est effectué par exemple à une température comprise entre 15 °C et 50 °C (de préférence entre 19 et 25°C), et dans des conditions d'humidité comprises entre 10% et 70%, et de préférence entre 50% et 60%, dans des conditions de pression atmosphérique standard.

L'angle de contact est alors mesuré 0,1 s après avoir posé une goutte d'eau sur la surface du film.

Le mouillage peut notamment être mesuré par tensiométrie à angle de contact, au moyen, par exemple, du tensiomètre DAT 1100, commercialisé par la société Fibro (Suède), ou à l'aide d'un appareil de mesure d'angle de contact usuel, tel que par exemple le SDT-200 commercialisé par la société IT Concept, utilisé en mode statique, ou en utilisant un MSA surface analyzer commercialisé par la société Krüss.

Dans un mode de réalisation préféré, le film formé après séchage par les compositions selon l'invention se caractérise en ce qu'il est hydrophile, et présente un angle de contact d'une goutte d'eau inférieure ou égal à 45°, de préférence inférieur ou égal à 40°, de préférence inférieur ou égal à 35°, encore plus préférablement inférieur ou égal à 30° et de manière préférée inférieur ou égal à 25°.

Dans un mode de réalisation préféré, les compositions selon l'invention ne comprennent pas d'agent ou de système filmogène autre que celui formé par l'acide spiculisporique et la base, tels que décrits plus haut. En particulier, les compositions selon l'invention comprennent moins de 5%, de préférence moins de 3%, de préférence moins de 2%, de préférence moins de 1% de préférence moins de 0,5%, et encore plus préférablement ne comprennent aucun agent ou système filmogène autre que celui formé par l'acide spiculisporique et la base, tels que décrits plus haut. Par « autre agent ou système filmogène », il est de préférence entendu des composés capables de former des films, seul, ou en association, et en particulier la pectine, les combinaisons de pectine et d'ions divalents, tels que le carbonate de calcium, ou les polymères fimogènes. Dans un mode de réalisation préféré, les compositions selon l'invention ne comprennent pas simultanément de la pectine et des ions divalents, tels que des sels de calsium et plus spécifiquement du carbonate de calcium. Dans un mode de réalisation préféré, les compositions selon l'invention sont exemptes de pectine. Dans un autre mode de réalisation préféré, les compositions selon l'invention sont exemptes de polymères fimogènes.

### Matières volatiles odorantes

La composition selon l'invention comprend avantageusement au moins une matière volatile odorante.

Par « matière volatile », on entend, au sens de l'invention, tout composé susceptible de s'évaporer au contact de la peau en moins d'une heure, à température ambiante et pression atmosphérique. La matière volatile est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1 300 Pa (0,01 à 10 mm de Hg).

Avantageusement ladite matière volatile est lipophile, elle est donc présente dans la phase grasse de l'émulsion. Parmi les matières volatiles odorantes, on entend essentiellement les huiles essentielles et les substances parfumantes. C'est-à-dire les substances parfumantes différentes des huiles essentielles.

### Huiles essentielles

Les huiles essentielles se distinguent des huiles végétales par le fait qu'elles ne peuvent pas être décomposées par saponification en glycérol et savon d'acide gras. Par ailleurs, elles sont volatiles.

Selon la définition donnée dans la norme internationale ISO 9235 et adoptée par la Commission de la Pharmacopée Européenne, une huile essentielle est un produit généralement de composition complexe, obtenu à partir d'une matière première végétale botaniquement définie, soit par entraînement à la vapeur d'eau, soit par distillation sèche, soit par extraction au moyen de solvants liquides ou gazeux, soit par un procédé mécanique approprié sans chauffage (expression à froid). L'huile essentielle est le plus souvent séparée de la phase aqueuse par un procédé physique n'entraînant pas de changement significatif de la composition. Ces huiles essentielles peuvent être également préparées par synthèse.

L'huile essentielle utilisée selon l'invention peut être obtenue à partir de tout matériel végétal issu de la plante entière ou de toute partie de ladite plante comme par exemple les feuilles, les tiges, les fleurs, les pétales, les graines, les fruits, les bourgeons, les racines, rameaux de plantes et/ou plantes entières.

L'huile essentielle utilisée selon l'invention peut être préparée selon les techniques mentionnées ci-dessus et sera de préférence obtenue selon la technique classique de l'entraînement à la vapeur d'eau.

Parmi les huiles essentielles utilisables selon l'invention, on peut citer celles obtenues à partir des plantes appartenant aux familles botaniques suivantes : Abiétaceés ou Pinacées : conifères ; Amaryllidacées ; Anacardiacées ; Anonacées : ylang ; Apiacées (par exemple les ombellifères) : aneth, angénique, coriandre, criste marine, carotte, persil ; Aracées ; Aristolochiacées ; Astéracées : achilée, armoise, camomille, hélichryse ; Bétulacées ; Brassicacées ; Burséracées : encens ; Caryophyllacées ; Canellacées ; Césalpiniacées : copaïfera (copahu) ; Chénopodacées ; Cistacées : ciste ; Cypéracées ; Diptérocarpacées ; Ericacées : gaulthérie (wintergreen) ; Euphorbiacées ; Fabacées ; Geraniacées : géranium ; Guttifères ; Hamamélidacées ; Hemandiacées ; Hypéricacées : millepertuis ; Iridacées ; Juglandacées ; Lamiacées : thym, origan, monarde, sarriette, basilic, marjolaines, menthes, patchouli, lavandes, sauges, cataire, romarin, hysope, mélisse, romarin ; Lauracées : ravensara, laurier, bois de rose, cannelle, litséa ; Liliacées : ail ; Magnoliacées : magnolia ; Malvacées ; Méliacées ; Monimiacées ; Moracées : chanvre, houblon ; Myricacées ; Mysristicacées : muscade ; Myrtacées : eucalyptus, tea tree, niaouli, cajeput, backousia, girofle, myrte; Oléacées ; Pipéracées : poivre ; Pittosporacées ; Poacées : citronnelle, lemongrass, vétiver ; Polygonacées ; Renonculacées ; Rosacées : roses ; Rubiacées ; Rutacées : tous les citrus ; Salicacées ; Santalacées : santal ; Saxifragacées ; Schisandracées ; Styracacées : benjoin ; Thymélacées : bois d'agar ; Tilliacées ; Valérianacées : valériane, nard ; Verbénacées : lantana, verveine ; Violacées ; Zingibéracées : galanga, curcuma, cardamome, gingembre ; Zygophyllacées.

On peut citer également les huiles essentielles extraites de fleurs (lis, lavande, rose, jasmin, Ylang-Ylang, néroli), de tiges et de feuilles (patchouli, géranium, petitgrain), de fruits (coriandre, anis, cumin, genièvre), d'écorces de fruits (bergamote, citron, orange), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes ( galbanum, élémi, benjoin, myrrhe, oliban, opopanax).

L'huile essentielle préférentiellement mise en oeuvre dans les compositions de l'invention est choisie parmi l'huile essentielle de géranium, l'huile essentielle de citronnelle, l'huile essentielle de cèdre, l'huile essentielle d'orange douce, l'huile essentielle d'origan vert, l'huile essentielle de lemongrass, l'huile essentielle de cataire citronnée, l'huile essentielle de romarin, l'huile essentielle de sarriette des Montagnes, l'huile essentielle de laser siler (apiacee graines ou feuille), l'huile essentielle de marjolaine, l'huile essentielle de buplevre (asteracee), l'huile essentielle d'achillée déodorante, l'huile essentielle de thym, l'huile essentielle de mélisse, l'huile essentielle de citron, l'huile essentielle d'eucalyptus, en particulier radiata ou globulus, l'huile essentielle de mandarine verte ou rouge, l'huile essentielle de girofle, l'huile essentielle de cannelle, ou leurs mélanges. De préférence, elle est choisie parmi l'huile essentielle de géranium, l'huile essentielle d'orange douce et l'huile essentielle d'origan vert.

La quantité d'huile essentielle va de 0,0001 % à 20 % en poids, de préférence de 0,001 % à 10 % avantageusement de 0,01% à 5% en poids par rapport au poids total de la composition.

### Huile essentielle de géranium

Selon un mode de réalisation préféré, la composition selon l'invention comprend de l'huile essentielle de géranium.

Le géranium est une plante herbacée appartenant à la famille des Géraniacées.

L'Huile essentielle de géranium est principalement disponible sous 2 variétés, à savoir le Pelargonium roseum asperum CV Bourbon et le Pelargonium roseum asperum CV Afrique du Nord (Egypte).

Leur nom INCI est identique, à savoir *Pelargonium graveolens.* Ainsi, on parle également d'huile essentielle de pélargonium. Dans le présent texte, les expressions « huile essentielle de géranium » et « huile essentielle de pélargonium » sont utilisées sans distinction de sens.

L'huile essentielle de géranium consiste essentiellement en l'association de 3 monoterpénoles, à savoir du citronellol (18 à 32 %), du géraniol (8 à 20 %) et du linalol (1,8 à 11 %), ainsi que des esters terpéniques correspondants.

L'huile essentielle de géranium est préparée par distillation classique par entraînement à la vapeur d'eau à partir des feuilles et des tiges de géranium.

L'entrainement à la vapeur correspond à la vaporisation, en présence de vapeur d'eau, d'une substance peu miscible à l'eau. La matière première est mise en présence d'eau portée à ébullition ou de vapeur d'eau dans un alambic. La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. On appelle « eau aromatique » ou « hydrolat » ou « eau distillée florale », le distillat aqueux qui subsiste à l'entraînement à la vapeur d'eau, une fois la séparation de l'huile essentielle effectuée.

A titre d'exemple d'huile essentielle de géranium selon la présente invention, on peut citer celle commercialisée par Elixens sous la dénomination PELARGONIUM GRAVEOLENS FLOWER OIL^{®}.

### Huile essentielle de citronnelle

Selon un mode de réalisation préféré, la composition selon l'invention comprend de l'huile essentielle de citronnelle.

La citronnelle est une grande herbe, originaire d'Inde et d'Asie du Sud-Est qui peut atteindre 1,50 mètre de haut. Elle se compose de feuilles étroites et lancéolées dont les pédoncules ressemblent à des branches. Son parfum, rappelant l'odeur du citron, est à l'origine du nom de la plante. Cette plante des zones tropicales, originaire de l'Inde et du Sri Lanka, pousse aussi en Afrique, Amérique du Sud, Amérique centrale et à Madagascar. Son huile essentielle est obtenue par distillation des feuilles hachées que l'on récolte plusieurs fois dans l'année.

Cette distillation s'effectue sans addition d'eau ou de vapeur d'eau dans une enceinte fermée conçue pour que le liquide soit récupéré dans sa partie basse.

A titre d'exemple d'huile essentielle de citronnelle selon la présente invention, on peut citer celle commercialisée par Elixens sous la dénomination CYMBOPOGON FLEXUOSUS OIL^{®}.

### Huile essentielle d'orange douce

Selon un mode de réalisation préféré, la composition selon l'invention comprend de l'huile essentielle de d'orange douce.

L'orange est le fruit de l'oranger (*Citrus sinensis*)*,* qui appartient à la famille des Rutacées. L'huile essentielle d'orange douce est obtenue de préférence par expression (pressage et grattage) des zestes d'orange. L'huile essentielle d'orange douce a comme nom INCI : CITRUS AURANTIUM DULCIS PEEL OIL.

Comme huile essentielle d'orange douce, on peut citer le produit CITRUS AURANTIUM DULCIS PEEL OIL commercialisé par la société ELIXENS.

Ce mode d'obtention ne s'applique généralement qu'aux fruits agrumes (Citrus spp.) par des procédés mécaniques à température ambiante. Le principe de la méthode est le suivant: les zestes sont dilacérés et le contenu des poches sécrétrices qui ont été rompues est récupéré par un procédé physique. Le procédé classique consiste à exercer sous un courant d'eau une action abrasive sur toute la surface du fruit. Après élimination des déchets solides, l'huile essentielle est séparée de la phase aqueuse par centrifugation. La plupart des installations industrielles permettent en fait la récupération simultanée ou séquentielle des jus de fruits et de l'huile essentielle.

### Huile essentielle d'origan vert

Selon un mode de réalisation préféré, la composition selon l'invention comprend de l'huile essentielle d'origan vert.

L'huile essentielle d'Origan vert, *Origanum heracleoticum L.,* est extraite des parties aériennes fleuries et se compose majoritairement de Phénols : carvacrol, thymol et de Monoterpènes : para-cymène et gamma-terpinène.

Cette huile essentielle est préconisée dans le cadre de traitement des états pelliculaires. Elle est anti-infectieuse à très large spectre d'action : antibactérienne, antivirale, antifongique et antiparasitaire très puissante. Elle procure un effet tonique général et possède en outre des propriétés immunostimulantes, mais également apéritives et digestives.

A titre d'exemple d'huile essentielle d'origan vert selon la présente invention, on peut citer celle commercialisée par Elixens sous la dénomination ORIGANUM HERACLEOTICUM FLOWER OIL^{®}.

### Huile essentielle de cèdre

Selon un mode de réalisation préféré, la composition selon l'invention comprend de l'huile essentielle de cèdre.

Le cèdre est un conifère (Cedrus) de la famille des Pinacées. L'huile essentielle de cèdre est obtenue de préférence par entraînement à la vapeur d'eau, méthode expliquée précédemment. L'huile essentielle de cèdre a comme nom INCI : CEDRUS ATLANTICA WOOD OIL.

A titre d'exemple d'huile essentielle de cèdre selon la présente invention, on peut citer celle commercialisée par Elixens sous la dénomination CEDRUS ATLANTICA WOOD OIL^{®}.

### Huile essentielle de cataire citronnée

Selon un mode de réalisation de l'invention, la composition selon l'invention comprend de l'huile essentielle de Cataire citronnée (*Nepeta cataria L. citriodora BECK*). Une telle huile essentielle convenant à l'invention peut être obtenue par extraction par distillation à la vapeur d'eau à partir des sommités fleuries. En particulier, une huile essentielle de Cataire citronnée comprend, principalement, un mélange de monoterpénols, de monoterpénals et de terpènes.

### Huile essentielle de romarin

Selon un mode de réalisation de l'invention, la composition conforme à l'invention comprend de l'huile essentielle de romarin.

Une huile essentielle de Romarin (*Rosmarinum Officinalis* Chemotype Cinéole ou *Rosmarinum Officinalis 'pyramidalis'*) convenant à l'invention peut être obtenue par extraction par distillation à la vapeur d'eau à partir des feuilles.

Une huile essentielle de Romarin type Afrique du Nord comprend, principalement, un mélange d'oxydes terpéniques, de monoterpénones, de monoterpènes, de monoterpénols, de sesquiterpènes, d'esters terpéniques, ainsi que de traces de verbénone, terpinolène, de γ-terpinène, de linalol, et de para-cymène.

### Huile essentielle de sarriette des Montagnes

Selon un mode de réalisation de l'invention, la composition selon l'invention comprend de l'huile essentielle de sarriette des montagnes.

Une huile essentielle de Sarriette des montagnes (*Satureja montana L., Satureja montana L. ssp montana*) convenant à l'invention peut être obtenue par extraction de la plante et des fleurs par distillation à la vapeur d'eau. Une huile essentielle de Sarriette des montagnes comprend, principalement, un mélange de phénols, de monoterpènes, de sesquiterpènes, d'oxydes terpéniques, de monoterpénols.

### Huile essentielle de thym

Selon un mode de réalisation de l'invention, la composition selon l'invention comprend de l'huile essentielle de thym.

Une huile essentielle de thym (*Thymus vulgaris* CT thymol) convenant à l'invention peut être obtenue par distillation à la vapeur d'eau des sommités fleuries de la plante. En particulier, une huile essentielle de thym à thymol comprend principalement des phénols (thymol et carvacrol) et des alcools (terpinène et bornéol)

### Huile essentielle de mélisse

Selon un mode de réalisation de l'invention, la composition selon l'invention comprend de l'huile essentielle de mélisse.

Une huile essentielle de Mélisse (*Melissa officinalis L.)* convenant à l'invention peut être obtenue par extraction des parties aériennes par distillation à la vapeur d'eau. Les parties aériennes sont préférentiellement récoltées de juin à septembre. En particulier, une huile essentielle de Mélisse comprend, principalement, un mélange d'aldéhydes, de sesquiterpènes, de monoterpènes, d'esters terpéniques, d'alcools, et de composés non volatiles.

### Huile essentielle de girofle

Selon un mode de réalisation de l'invention, la composition conforme à l'invention comprend de l'huile essentielle de girofle.

Une huile essentielle de Clou de Girofle (*Eugenia caryophyllus ou E. aromatica Syzygium aromaticum*) convenant à l'invention peut être obtenue par extraction par distillation à la vapeur d'eau à partir du Clou (bouton floral). En particulier, une huile essentielle de Clou de Girofle comprend, principalement, un mélange de phénol, de sesquiterpènes et d'esters.

### Huile essentielle de cannelle

Selon un mode de réalisation de l'invention, la composition conforme à l'invention comprend de l'huile essentielle de cannelle.

Une huile essentielle de cannelle (*Cinnamomum cassia*) convenant à l'invention peut être obtenue par distillation à la vapeur d'eau de l'écorce de l'arbre. En particulier, une huile essentielle de cannelle comprend, principalement un aldéhyde aromatique, le cinnamaldéhyde ainsi que des phénols tels que le chavicol et l'isoeugénol.

### Huile essentielle de citron

Selon un mode de réalisation préféré, la composition selon l'invention comprend de l'huile essentielle de citron.

Une huile essentielle de citron (*Citrus limonum* L.) est obtenue de préférence par expression (pressage et grattage) des zestes de citron.

### Huile essentielle d'eucalyptus

Selon un mode de réalisation préféré, la composition selon l'invention comprend de l'huile essentielle d'eucalyptus, de préférence radiata ou globulus.

Une huile essentielle d'eucalyptus (Eucalyptus radiata LABILL) convenant à l'invention peut être obtenue par distillation à la vapeur d'eau des feuilles de l'arbre.

### Huile essentielle de mandarine verte ou rouge

Selon un mode de réalisation préféré, la composition selon l'invention comprend de l'huile essentielle de mandarine verte ou rouge.

Une huile essentielle de mandarine verte (*Citrus reticulata blanco*) est obtenue de préférence par expression (pressage et grattage) des zestes de mandarine

### Substances parfumantes

La matière volatile odorante peut également être choisie parmi les substances parfumantes, ou parfums, différentes des huiles essentielles.

Les parfums sont des compositions contenant notamment les matières premières décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, Ill.

Il peut également s'agir de produits naturels, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Des exemples de substances parfumantes sont notamment : le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol, l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha - hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

Selon un mode préféré de réalisation de l'invention, on utilise un mélange de différentes substances parfumantes qui engendrent en commun une note plaisante pour l'utilisateur.

On choisira de préférence les substances parfumantes de telles sorte qu'elles produisent des notes (tête, coeur et fond) dans les familles suivantes : les hespéridés, les aromatiques, les notes florales, les épicées, les boisées, les gourmands, les chyprés, les fougères, les cuirés et les muscs.

La quantité de substance(s) parfumante(s) est généralement d'au moins 0,01 % en poids, de préférence au plus 30 % en poids, en particulier de 0,1 % à 10 % en poids, de préférence de 0,3% à 10%, et notamment de 0,1 % à 5 % ou de 0,3 % à 5% par rapport au poids total de la composition.

### Phase aqueuse

L'eau peut être présente en une teneur totale allant de de 30 à 90 %, de préférence 40 à 85 % en poids, par rapport au poids total de la composition.

De façon préférée, la composition selon l'invention comprend au moins 30 %, de préférence au moins 40 % en poids d'eau, de préférence au moins 50 % en poids d'eau, par rapport au poids total de la composition.

La composition conforme à l'invention peut comprendre outre de l'eau, au moins un solvant hydrosoluble.

La phase aqueuse constitue la phase continue de la composition.

Par composition à phase continue aqueuse, on entend que la composition présente une conductivité, mesurée à 25 °C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

L'eau utilisée dans la composition de l'invention peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée). Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

L'eau utilisée dans la composition de l'invention peut également être une eau préparée à partir d'un végétal, comme une " eau d'une plante " lorsqu'il est obtenu d'une plante entière ou d'une partie de celle-ci, ou " eau florale " lorsque cette dernière est obtenue à partir de fleurs. De telles eaux obtenues à partir d'un végétal (ou toute matière première végétale botaniquement définie), notamment d'un végétal en entier et/ou d'une partie d'un végétal, sont également appelées « hydrolat ».

On entend par hydrolat, un distillat aqueux obtenu à partir d'une matière première végétale par entraînement à la vapeur d'eau. L'entrainement à la vapeur d'eau correspond à la vaporisation en présence de vapeur d'eau d'une substance peu miscible à l'eau. L'hydrolat selon l'invention peut être un distillat aqueux, obtenu à partir d'un végétal en entier et/ou d'une partie de ce végétal, qui subsiste après l'entraînement à la vapeur d'eau, une fois la séparation de l'huile essentielle effectuée. La matière première peut être mise en présence d'eau portée à ébullition ou de vapeur d'eau dans un alambic. La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. L'" hydrolat " est ainsi le distillat aqueux qui subsiste à l'entraînement à la vapeur d'eau, une fois la séparation de l'huile essentielle effectuée. Un hydrolat selon l'invention peut être également appelé " eau aromatique ".

L'hydrolat peut être obtenu à partir d'un végétal en entier, de préférence une plante, un arbuste ou une fleur, ou d'une partie de ce végétal choisie parmi les fleurs, feuilles, tiges, graines, fruits, racines, pétales, bourgeons, qui peuvent être à divers états de siccité (forme sèche, flétrie, fraîche), et leurs mélanges.

Particulièrement dans le cadre la présente invention, l'hydrolat est un hydrolat d'un végétal et/ou d'une partie d'un végétal choisi parmi la famille Rosaceae, de préférence genre Rosa, la famille Asteraceae, de préférence genre Centaurea et/ou genre Chamaemelum, la famille Lamiaceae, de préférence genre Lavandula, de la famille Rutaceae, de préférence genre Citrus, la famille Lamiaceae, de préférence le genre Melissa et/ou le genre Mentha, de la famille Verbenaceae, de préférence genre Aloysia, et leurs mélanges.

Plus particulièrement dans le cadre la présente invention, l'hydrolat est un hydrolat d'un végétal et/ou d'une partie d'un végétal choisi parmi Rosa Damascena, Centaurea Cyanus, Anthémis Nobilis ou Chamaemelum nobile, Lavandula Angustifolia, Citrus Aurantium Amara, Melissa Officinalis, Mentha Piperrita, Lippia Citriodora, et leurs mélanges.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol.

Dans la présente invention, par « polyol » on entend les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄, ou les polyols tels que par exemple la glycérine.

Comme déjà mentionné, la composition selon l'invention comprend moins de 3 %, de préférence moins de 1 % en poids de glycol(s) ou de polyol(s) par rapport au poids total de la composition. Avantageusement la composition selon l'invention est dénuée de glycol(s) et/ou de polyol(s).

Avantageusement le ratio glycol(s)/acide spiculisporique ou polyol(s)/acide spiculisporique est inférieur à 1, de préférence inférieur à 0,2.

La phase aqueuse (eau et éventuellement le solvant miscible à l'eau) peut être présente dans la composition en une teneur allant de 0,1 % à 99,9 % en poids, par rapport au poids total de la composition, de préférence allant de 40 % à 95 % en poids.

### Agent épaississant

Une composition selon l'invention peut comprendre au moins un agent gélifiant hydrophile en tant qu'épaississant.

De préférence les gélifiants hydrophiles sont polymériques et avantageusement ils sont naturels ou d'origine naturelle.

Au sens de l'invention, l'expression « *d'origine naturelle* » entend désigner les gélifiants polymériques obtenus par modification des gélifiants polymériques naturels.

Ces gélifiants peuvent être particulaires ou non particulaires.

Plus précisément, ces gélifiants relèvent de la catégorie des polysaccharides.

De manière avantageuse, les polysaccharides peuvent être choisis parmi les carraghénanes, en particulier la kappa-carraghenane, la gomme de gellane, l'agar-agar, la gomme de xanthane, les composés à base d'alginate, en particulier l'alginate de sodium, la gomme de scéroglucane, la gomme de guar, l'inuline, le pullulan, et leurs mélanges.

De manière préférée, le gélifiant hydrophile est la gomme de xanthane.

En fonction de l'application visée, une composition selon l'invention peut comprendre en outre une ou plusieurs charge(s) classiquement utilisée(s) dans les compositions notamment de soin et/ou de maquillage, des matières colorantes notamment des pigments, des nacres, des particules à reflet métallique.

Un avantage de la présente invention est qu'elle permet une dispersion homogène de ces charges, nacres, pigments dans la composition.

Bien entendu l'homme du métier choisira ces additifs de manière à ce qu'ils soient compatibles avec les autres composants de la composition.

### Charge

Une composition selon l'invention peut comprendre en outre une ou plusieurs charge(s) classiquement utilisée(s) dans les compositions notamment de soin et/ou de maquillage et différentes des pigments, nacres, particules à reflet métallique.

Ces charges sont des particules incolores ou blanches, solides de toutes formes, qui se présentent sous une forme insoluble et dispersée dans le milieu de la composition.

De nature minérale ou organique, naturelle ou synthétique, elles permettent de conférer à la composition les contenant de la douceur, de la matité et de l'uniformité au maquillage. En outre, ces charges permettent avantageusement de lutter contre différentes agressions telles que le sébum ou la sueur.

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

En particulier, de telles charges peuvent être présentes dans une composition selon l'invention dans une teneur comprise entre 0.01 % et 25 % en poids, notamment comprise entre 0.1 % et 20 % en poids, en particulier comprise entre 1 % et 10 %, poids, par rapport au poids total de la composition

### Nacres

La composition selon l'invention peut également comprendre au moins une nacre.

Par « *nacres* », il faut comprendre des particules colorées de toute forme, irisées ou non, notamment produites par certains mollusques dans leur coquille ou bien synthétisées, et qui présentent un effet de couleur par interférence optique.

Une composition selon l'invention peut comprendre de 0 % à 15 % en poids de nacres, par rapport au poids total de ladite composition.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres Timica, Flamenco et Duochrome (sur base de mica) commercialisées par la société ENGELHARD, les nacres Timiron commercialisées par la société Merck, les nacres sur base de mica Prestige commercialisées par la société Eckart et les nacres sur base de mica synthétique Sunshine commercialisées par la société Sun Chemical.

Les nacres peuvent plus particulièrement posséder une couleur ou un reflet jaune, rose, rouge, bronze, orangé, brun, or et/ou cuivré.

De manière avantageuse, les nacres conformes à l'invention sont les micas recouverts de dioxyde de titane ou d'oxyde de fer ainsi que l'oxychlorure de bismuth.

### Pigments

Comme énoncé précédemment, une composition peut contenir en outre des pigments.

Ces pigments peuvent être des pigments minéraux notamment tel que définis précédemment.

Ces pigments peuvent être également des pigments organiques.

Par « *pigment organique* », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque. Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).

A titre d'exemples de laques, on peut citer le produit connu sous la dénomination D&C Red 7 (CI 15 850:1).
la composition selon l'invention comprend en outre au moins un filtre UV et optionellement un additif choisi parmi les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

Comme indiqué précédemment, une composition selon l'invention comprend au moins un filtre UV, ledit filtre UV étant en particulier liphophile.

### Filtre UV

Ce filtre est choisi parmi les filtres UV-A et/ou UV-B.

Encore plus particulièrement, ledit filtre UV est choisi parmi les filtres UV organiques hydrosolubles, les filtres organiques liposolubles et leurs mélanges et plus particulièrement les filtres UV organiques liposolubles.

A titre d'illustration des filtres UV et de façon non limitative, on peut citer les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40^{®} disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique, et préférentiellement le 4-méthylbenzylidène camphre (Eusolex 6300^{®} disponible chez Merck) ; les benzimidazoles, en particulier le benzimidazilate (Neo Heliopan AP^{®} disponible chez Haarmann et Reimer), ou l'acide phénylbenzimidazole sulfonique (Eusolex 232^{®} disponible chez Merck) ; les benzotriazoles, en particulier le drométrizole trisiloxane, ou le méthylène bis-benzotriazolyltétraméthylbutylphénol (Tinosorb M^{®} disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35^{®} disponible chez B.A.S.F.), l'octylméthoxycinnamate (Parsol MCX^{®} disponible chez Hoffmann La Roche), ou l'octocrylène (Uvinul 539^{®} disponible chez B.A.S.F.) ; les dibenzoylméthanes, en particulier le butyl méthoxydibenzoylméthane (Parsol 1789^{®}) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB^{®} disponible chez 3V Sigma), l'éthylhexyltriazone (Uvinul T150^{®} disponible chez B.A.S.F.), ou l'éthyl PABA (benzocaïne) ; le Mexoryl^{®} ; les salicylates, en particulier le salicyclate de dipropylèneglycol, le salicylate d'éthylhexyle, l'homosalate, ou le TEA salicylate ; les triazines, en particulier l'anisotriazine (Tinosorb S^{®} disponible chez Ciba) ; le drometrizole trisiloxane, l'oxyde de zinc, le dioxyde de titane, l'oxyde de zinc, de fer, de zirconium, de cérium enrobés ou non.

Dans un mode de réalisation préféré, la présence d'acide spiculisporique dans les compositions selon l'invention comprenant des filtres de protection solaire résulte en une augmentation surprenante du SPF par rapport à des compositions équivalentes ne comprenant pas d'acide spiculisporique.

### Agent dispersant additionnel

Avantageusement, une composition selon l'invention peut comprendre en outre un agent dispersant additionnel.

Un tel agent dispersant peut être un tensioactif, un oligomère, un polymère ou un mélange de plusieurs d'entre eux.

Lorsque la composition selon l'invention comprend un tensioactif additionnel, cet dernier est de préférence présent en une teneur inférieure à 5%, de préférence inférieure à 3%, de préférence inférieure à 2%, de préférence inférieure à 1%, de préférence inférieure à 0,5% en poids par rapport au poids total de la composition. Dans un mode de réalisation préféré, la composition selon l'invention ne contient pas de tensioactif additionnel.

Dans un autre mode de réalisation préféré, la composition selon l'invention ne comprend pas de tensioactif sulfaté et/ou sulfonaté, tel que par exemple ceux décrits dans la demande WO2015067785.

Selon un mode de réalisation particulier, un agent dispersant conforme à l'invention est un tensioactif.

### Actif

Selon un mode de réalisation particulier, la composition selon l'invention peut en outre comprendre au moins un actif additionnel choisi parmi les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants, les parfums et leurs mélanges.

Ces actifs additionnels peuvent être présents dans la composition selon l'invention en une teneur allant de 0,001 % à 30 % en poids, de préférence de 0,01 % à 20 % en poids, et plus préférentiellement de 0,1 % à 15 % en poids, par rapport au poids total de la composition les comprenant.

### Agents anti-rides ou anti-âge

A titre représentatif d'agents anti-rides ou anti-âge utilisables dans la présente invention peuvent plus particulièrement être mentionnés l'adénosine, le rétinol et ses dérivés, l'acide ascorbique et ses dérivés, tels que l'ascorbyl phosphate de magnésium et le glucoside d'ascorbyle ; le tocophérol et ses dérivés, tels que l'acétate de tocophéryle ; l'acide nicotinique et ses précurseurs, tels que la nicotinamide ; l'ubiquinone ; le glutathion et ses précurseurs, tels que l'acide L-2-oxothiazolidine-4-carboxylique ; les composés C-glycoside et leurs dérivés, tels que décrits notamment ci-après ; les extraits de plantes et notamment les extraits de criste marine et de feuille d'olivier, ainsi que les protéines végétales et leurs hydrolysats, tels que les hydrolysats de protéines de riz ou de soja ; ou encore les extraits de graines de *Vigna aconitifolia* comme ceux commercialisés par la société Cognis sous les références Vitoptine LS9529 et Vit-A-Like LS9737 ; les extraits d'algues et en particulier de laminaires ; les extraits bactériens ; les sapogénines, telles que la diosgénine et les extraits de Dioscorées, en particulier de Wild Yam, en contenant ; les α-hydroxyacides ; les β-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène ; les sels de manganèse et de magnésium, en particulier les gluconates ; et leurs mélanges.

### Agents humectants ou hydratants

Comme agents humectants ou hydratants, on peut citer notamment un extrait d'aloe vera, l'urée et ses dérivés notamment l'Hydrovance^{®} commercialisée par National Starch, des monosaccharides comme le mannose, l'acide hyaluronique, les AHA, les BHA, des homopolymères d'acide acrylique comme le Lipidure-HM^{®} de NOF corporation, le beta-glucan et en particulier le sodium carboxymethyl beta-glucane de Mibelle-AG-Biochemistry ; un polyoxybutylène polyoxyéthylène polyoxypropylène glycérol comme le WILBRIDE S-753L^{®} de NOF corporation, une huile de rosier muscat commercialisée par Nestlé ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon.

### Agents desquamants additionnels

Comme agents desquamants, on pourra citer les beta-hydroxyacides, en particulier l'acide salicylique et ses dérivés autres que l'acide n-octanoyl 5-salicylique ; l'urée ; les acides glycolique, citrique, lactique, tartrique, malique ou mandélique ; l'acide 4-(2-hydroxyéthyl)piperazine-1-propanesulfonique (HEPES) ; l'extrait de Saphora japonica ; le miel ; le N-acétyl glucosamine ; le méthyl glycine diacétate de sodium, les alpha-hydroxy acides (AHA), les Beta-hydroxy acides (BHA), et leurs mélanges.

### Agents antioxydants

Comme agents antioxydants, on peut plus particulièrement citer le tocophérol et ses esters, en particulier l'acétate de tocophérol ; l'EDTA, l'acide ascorbique et ses dérivés, en particulier l'ascorbyl magnésium phosphate et l'ascorbyl glucoside ; les chélatants, tels que le BHT, le BHA, le N,N'-bis(3,4,5-triméthoxybenzyl) éthylenediamine et ses sels, et leurs mélanges.

### Agents dermodécontractants ou dermorelaxants

Comme agents dermodécontractants ou dermorelaxants, on peut citer tout particulièrement le gluconate de manganèse, le wild yam, la criste marine, la glycine et l'alvérine.

### Actifs stimulants la synthèse de macromolécules dermiques et/ou épidermiques et/ou empêchant leur dégradation

Comme actifs stimulant la synthèse de macromolécules dermiques et/ou épidermiques et/ou empêchant leur dégradation, on peut citer : les peptides extraits de végétaux, tels que l'hydrolysat de soja commercialisé par la société BASF Beauty Care Solutions sous la dénomination commerciale Phytokine^{®} l'extrait de malt tel que commercialisé sous la dénomination Collalift^{®} par la société BASF BCS; les peptides de riz tel que le Nutripeptide^{®} de SILAB, ou encore un extrait de peptides de riz tel que la Colhibin^{®} de Pentapharm DSM, le méthylsilanol mannuronate tel que l'Algisium C^{®} commercialisé par Exsymol ; un extrait de vaccinium myrtillus tel que ceux décrits dans la demande FR-A-2 814 950 ; l'extrait de lupin commercialisé par la société SILAB sous la dénomination commerciale Structurine^{®}, et leurs mélanges, l'hydrolat de verveine.

### Agents dépigmentants

Comme agents dépigmentants, on peut citer les céramides, la vitamine C et ses dérivés et notamment la vit CG, CP et 3-O éthyl vitamine C, l'alpha et la béta arbutine, l'acide férulique, l'acide kojique, le résorcinol et ses dérivés, le D calcium panthéteine sulfonate, l'acide lipoique, l'acide ellagique, la vitamine B3, le phényléthyl résorcinol comme le Symwhite 377^{®} de la société Symrise, une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B^{®}, un extrait de sucre brun (Saccharum officinarum), tel que l'extrait de mélasse commercialisé par la société Taiyo Kagaku sous la dénomination Molasses Liquid, un mélange d'acide undecylenique et phénylalanine undecylenoyl, tel que le Sepiwhite MSH^{®} de Seppic.

### Agents déodorants

Comme agents déodorants conformes à l'invention, on peut citer les huiles essentielles, des agents bactériostatiques ou des agents bactéricides agissant sur les germes des odeurs axillaires, comme le 2,4,4'-trichloro-2'-hydroxydiphényléther (^{®}Triclosan), le 2,4-dichloro-2'-hydroxydiphényléther, le 3',4',5'-trichlorosalicylanilide, la 1-(3',4'-dichlorophenyl)-3-(4'-chlorophenyl)urée (^{®}Triclocarban) ou le 3,7,11-triméthyldodéca-2,5,10-triénol (^{®}Farnesol).

Des agents déodorants conformes à l'invention peuvent également être choisis parmi les sels d'ammonium quaternaires comme les sels de cetyltrimethylammonium, les sels de cétylpyridinium , le DPTA (acide 1,3-diaminopropanetétraacétique), le 1,2 decanediol (Symclariol de la société Symrise) ; les dérivés de glycérine comme par exemple le Caprylic/Capric Glycerides (CAPMUL MCM de Abitec), le caprylate ou caprate de Glycerol (DERMOSOFT GMCY et DERMOSOFT GMC respectivement de STRAETMANS), le Polyglyceryl-2 Caprate (DERMOSOFT DGMC de STRAETMANS) ; les dérivés de biguanide comme les sels de polyhexaméthylène biguanide ; la chlorhexidine et ses sels; et le 4-Phenyl-4,4-dimethyl-2butanol (SYMDEO MPP de Symrise).

Peuvent encore être cités comme agents déodorants les sels de zinc comme le salicylate de zinc, le gluconate de zinc, le pidolate de zinc ; le sulfate de zinc, le chlorure de zinc, le lactate de zinc, le phénolsulfonate de zinc ou l'acide salicylique et ses dérivés tels que l'acide n-octanoyl-5-salicylique.

En outre, des agents déodorants selon l'invention peuvent être choisis parmi les absorbeurs d'odeur comme les ricinoléates de zinc, le bicarbonate de sodium ; les Zéolithes métalliques ou non, les cyclodextrines.

Il peut s'agit également d'un agent chélatant tel que la DISSOLVINE GL-47-S de Akzo Nobel, EDTA ; DPTA.

Il peut encore s'agir d'inhibiteur enzymatique tel que le triéthyle citrate.

En cas d'incompatibilité ou pour les stabiliser, certains des agents mentionnés ci-dessus peuvent être incorporés dans des sphérules, notamment des vésicules ioniques ou non-ioniques et/ou des particules (capsules et/ou sphères).

Bien entendu, les agents déodorants pouvant être présents dans une composition conforme à l'invention ne doivent pas altérer les propriétés avantageuses de la composition indiquées précédemment.

La composition selon l'invention peut avoir la forme d'un produit de soin ou de maquillage du visage et/ou du corps, et être conditionnée par exemple sous forme de crème en pot ou de fluide en tube ou en flacon pompe.

Il relève des opérations de routine de l'homme de l'art d'ajuster la nature et la quantité des additifs présents dans les compositions conformes à l'invention, de telle sorte que les propriétés cosmétiques désirées de celles-ci n'en soient pas affectées.

Selon un autre mode de réalisation, une composition de l'invention peut avantageusement se présenter sous la forme d'une composition de base de maquillage pour le maquillage.

Selon un autre mode de réalisation, une composition de l'invention peut avantageusement se présenter sous la forme d'un fond de teint.

De telles compositions sont notamment préparées selon les connaissances générales de l'homme de l'art.

Dans toute la description, y compris les revendications, l'expression « *comportant un* » doit être comprise comme étant synonyme de « *comportant au moins un* », sauf si le contraire est spécifié.

Les expressions « *compris entre* ... *et* ... » et « *allant de* ... *à* ... » doivent se comprendre bornes incluses, sauf si le contraire est spécifié.

L'invention est illustrée plus en détail par les exemples présentés ci-après. Sauf indication contraire, les quantités indiquées sont exprimées en pourcentage massique.

### EXEMPLES

Dans les tableaux suivants, la quantité de chaque composé est donnée en pourcentage en poids de matière active par rapport au poids total de la composition.

### Exemple 1 :

### Préparation des compositions

Les compositions sont préparées au Rayneri selon le protocole suivant :
- l'eau est pesée dans le becher de préparation,
- l'acide spiculisporique est introduit sous agitation mécanique, étant insoluble dans l'eau, il reste sous forme de poudre,
- la base notamment l'arginine est ajoutée sous agitation mécanique, le mélange des poudres se dissous au fur et à mesure, la solution devient totalement translucide après 5min d'agitation,
- la ou les huiles sont introduite(s) et le filtre UV, toujours sous agitation et l'agitation est maintenue 10 minutes à 700-1000 RPM en fonction de la viscosité du milieu.
- Le conservateur est ajouté ainsi que de l'eau qui permet d'atteindre la concentration visée.

### Préparations des films

Les films sont préparés à l'aide d'un tire-film qui se présente sous la forme d'un bras mécanique qui balaie une surface plane à une vitesse contrôlée.

L'épaisseur des films est contrôlée à l'aide d'éprouvettes d'épaisseurs de fentes précises. Le film est ensuite laissé à l'air libre afin de sécher et le comportement au séchage est observé. Le comportement au séchage reflète la façon dont le tensio-actif structure la phase grasse au séchage ainsi que la résistance du film obtenu à l'humidité ambiante.

### Série 1 -Emulsions comprenant un mono-sel d'arginine de l'acide spiculisporique

**Tableau 1**

| | | **Ex 1 conforme** | **Ex A comparatif** | **Ex B comparatif** | **Ex 2 conforme** | **Ex C comparatif** |
|---|---|---|---|---|---|---|
| A | Acide spiculisporique | 5 | 5 | 0 | 1 | 5 |
| | Arginine | 2,9 | 2,9 | 0 | 0,6 | 0 |
| | eau | 50 | 50 | 50 | 50 | 50 |
| | Hydroxyde de potassium | 0 | 0 | 0 | 0 | 1,9 |
| B | Huile de jojoba | 5 | 5 | 5 | 5 | 5 |
| | Octyldodecanol | 10 | 10 | 10 | 10 | 10 |
| | Oxyde de dioctyle stabilisé | 5 | 5 | 5 | 5 | 5 |
| | ETHYLHEXYL METHOXYCINNAMATE, commercialisé sous le nom PARSOL MCX par DSM NUTRITIONAL PRODUCTS | 1 | 1 | 1 | 1 | 1 |
| C | glycérine | 0 | 5 | 0 | 0 | 0 |
| D | xanthane | 0,4 | 0,4 | 1 | 0,4 | 0,4 |
| E | Conservateur | 0,5 | 0,5 | 0,5 | 0,5 | 0,5 |
| | eau | Qsp 100 | Qsp 100 | Qsp 100 | Qsp 100 | Qsp 100 |

### Résultats

### Exemples conformes

L'exemple **1** comprenant 5 % de mono-sel d'arginine de l'acide spiculisporique conduit, après séchage, à la formation d'un film homogène et stable. Le film reste stable après stockage pendant deux semaines à l'air libre et à température ambiante. En effet on n'observe pas de coalescence de l émulsion ni de séparation des phases. Le film obtenu est sec et « rigide » en ce sens qu'il peut être manipulé.

L'exemple **2** comprenant 1 % de mono-sel d'arginine de l'acide spiculisporique conduit, après séchage, à la formation d'un film homogène et stable. Le film est stable après stockage pendant deux semaines à l'air libre et à température ambiante. En effet on n'observe pas de coalescence de l émulsion ni de séparation des phases.

### Exemples comparatifs

L'exemple **A** comprenant 5 % de mono-sel d'arginine de l'acide spiculisporique et 5 % de glycérine conduit, après séchage, à la formation d'un film nonhomogène qui comporte des trous. La stabilité du film est mauvaise. Le film devient rapidement gras, collant, il ne peut pas être manipulé.

Au bout d'une heure, on observe une nette séparation des phases de l'émulsion.

Les mêmes résultats ont été observés en remplaçant les 5 % de glycérine par 5 % de propanediol.

L'émulsion de l'exemple **B,** sans acide spiculisporique, comprenant 1 % de xanthane n'est pas stable, on observe un déphasage rapidement après la préparation. Par ailleurs cette émulsion ne conduit pas à un film homogène et stable. Cet exemple montre que le xanthane seul ne permet pas l'obtention d'une composition présentant des propriétés filmogènes.

L'émulsion de l'exemple **C** comprenant 5 % de mono-sel de potassium de l'acide spiculisporique est stable après la préparation mais au bout de 10 heures, l'émulsion présente des cristaux. Le film obtenu après séchage présente rapidement un déphasage.

### Série 2 -Emulsions comprenant un sel d'arginine de l'acide spiculisporique- variation de la phase grasse

**Tableau 2**

| | | **Ex 3 conforme** | **Ex 4 conforme** | **Ex 5 conforme** | **Ex 6 conforme** |
|---|---|---|---|---|---|
| A | Acide spiculisporique | 5 | 5 | 5 | 5 |
| | Arginine | 2,9 | 2,9 | 2,9 | 2,9 |
| | eau | 50 | 50 | 50 | 50 |
| B | Octyldodecanol | 20 | 0 | 0 | 0 |
| | Oxyde de dioctyle stabilisé | 0 | 20 | 0 | 0 |
| | Squalane | 0 | 0 | 20 | 0 |
| | Huile d'olive | 0 | 0 | 0 | 20 |
| | ETHYLHEXYL METHOXY CINNAMATE, commercialisé sous le nom PARSOL MCX par D SM NUTRITIONAL PRODUCTS | 1 | 1 | 1 | 1 |
| C | xanthane | 0,4 | 0,4 | 0,4 | 0,4 |
| E | Conservateur | 0,5 | 0,5 | 0,5 | 0,5 |
| | eau | Qsp 100 | Qsp 100 | Qsp 100 | Qsp 100 |

### Résultats

Des émulsions ont été préparées en utilisant des huiles de polarité très différentes : de la moins polaire (squalane) à la plus polaire (huile d'olive). Dans tous les cas, des émulsions stables ont été obtenues conduisant à des films de bonne qualité.

### Série 3 -Emulsions comprenant un sel d'arginine de l'acide spiculisporique- variation du taux de phase grasse

**Tableau 3**

| | | **Ex 1 conforme** | **Ex 7 conforme** | **Ex 8 conforme** |
|---|---|---|---|---|
| A | Acide spiculisporique | 5 | 5 | 5 |
| | Arginine | 2,9 | 2,9 | 2,9 |
| | eau | 50 | 30 | 20 |
| B | Huile de jojoba | 5 | 10 | 15 |
| | Octyldodecanol | 10 | 20 | 30 |
| | Oxyde de dioctyle stabilisé | 5 | 10 | 15 |
| | ETHYLHEXYL METHOXYCINNAMATE, commercialisé sous le nom PARSOL MCX par DSM NUTRITIONAL PRODUCTS | 1 | 1 | 1 |
| D | xanthane | 0,4 | 0,4 | 1 |
| E | Conservateur | 0,5 | 0,5 | 0,5 |
| | eau | Qsp 100 | Qsp 100 | Qsp 100 |

### Résultats

Toutes les émulsions H/E obtenues, même à fort taux de phase grasse, sont stables et conduisent à des films de bonne qualité : ces films sont homogènes, présentent une bonne tenue, et un toucher sec et non gras.

### Série 4 -Emulsions comprenant des polymères filmogènes ou un tensioactif synthétique

**Tableau 4**

| | | **Ex D comparatif** | **Ex E comparatif** | **Ex F comparatif** |
|---|---|---|---|---|
| A | Lauroyle Glutamate de Sodium (SLG) | 5 | 0 | 0 |
| | eau | 50 | 30 | 20 |
| B | Huile de jojoba | 5 | 5 | 5 |
| | Octyldodecanol | 10 | 10 | 10 |
| | Oxyde de dioctyle stabilisé | 5 | 5 | 5 |
| | ETHYLHEXYL METHOXYCINNAMATE, commercialisé sous le nom PARSOL MCX par DSM NUTRITIONAL PRODUCTS | 1 | 1 | 1 |
| D | xanthane | 0,4 | 0 | 0 |
| | Alginate | 0 | 2 | 0 |
| | Copolymère acide acrylique /mathcrylate de stearyle | 0 | 0 | 1,4 |
| | 2amino 2méthyl 1-propanol | 0 | 0 | 0.8 |
| E | Conservateur | 0,5 | 0,5 | 0,5 |
| | eau | Qsp 100 | Qsp 100 | Qsp 100 |

### Résultats

Dans la composition de l'exemple comparatif D sont utilisés du SLG, (tensioactif synthétique) et du xanthane pour stabiliser l'émulsion formée et pouvoir préparer un film. Le film ainsi obtenu s'étale bien mais n'est pas stable après séchage puisqu'on observe de la coalescence.

Dans chacune des compositions des exemples comparatifs E et F, un polymère filmogène est utilisé, ces polymères ne permettent pas de faire des émulsions stables et les films obtenus sont inhomogènes. Les polymères testés ne permettent pas de stabiliser un film gras.

### Série 4 -Caractérisation du film formé par les émulsions d'acide spiculisporique selon l'invention

Les films formés après séchage d'émulsions selon l'invention comprenant de l'acide spiculisporique ont été caractérisés par un analyseur de surface par mesure de l'angle de contact avec une goutte d'eau.

La mesure de l'angle a été effectuée avec un analyseur de surface MSA (Krüss). Les formules ont été appliquées sur un support de type skinFX (Support à base de silicone d'un diamètre de 80 mm, recouvert d'un film de polyuréthane) et laissées sécher pendant une durée de 20 minutes. Une goutte d'eau est ensuite déposée sur la surface du film et visualisée avec une caméra.

L'angle de contact est mesuré et reporté dans un tableau.

Différentes émulsions sont évaluées, en faisant varier la concentration d'acide spiculisporique, la polarité de l'huile, la nature des mélanges d'huils et la teneur en huiles. Les émulsions testées sont listées dans le tableau ci-dessous.

| Formule | Emulsion G (Conforme) | Emulsion H (conforme) | Emulsion I (conforme) | Emulsion J (conforme) | Emulsion K (conforme) |
|---|---|---|---|---|---|
| Acide spiculisporique | 5 | 1 | 1 | 1 | 1 |
| Arginine | 1,1 | 1,1 | 1,1 | 1,1 | 1,1 |
| octyldodecanol | 20 | 20 | 40 | 60 | 0 |
| Huile de coco | 0 | 0 | 0 | 0 | 60 |
| xanthane | 0,4 | 0,4 | 0,4 | 0,4 | 0,4 |
| ETHYLHEXYL METHOXYCINNAMATE, commercialisé sous le nom PARSOL MCX par DSM NUTRITIONAL PRODUCTS | 1 | 1 | 1 | 1 | 1 |
| Eau | Qsp 100 | Qsp 100 | Qsp 100 | Qsp 100 | Qsp 100 |
| CA(M) [°] | 24,49 | 21,19 | 17,17 | 17,47 | 15,37 |
| ± | 13,98 | 1,09 | 4,29 | 3,62 | 2,59 |

Les résultats obtenus montrent que quelque soit la nature de la phase huileuse, sa concentration ou la concentration de l'acide spiculisporique, l'angle de contact du film formé avec la goutte d'eau ne change pas de manière significative (la mesure de l'angle effectuée sur la peau skin FX seule, c'est à dire non recouverte d'un film selon la présente invention est de 123,26 CA(M) [°] ±2,42. Le film formé par chacune des émulsions G à K est de nature hydrophile. La formation de tels films hydrophiles présente un fort intérêt pour la mise en oeuvre dans des applications cométiques variées, tells que l'anti-pollution l'hydratation, le maquillage ou le soin capillaire.

### Exemple 2 : Compositions comprenant des huiles essentielles

### Préparation des compositions

Les compositions suivantes sont préparées comme décrit plus haut à l'exemple 1.

| | | Ex 1 comparatif | Ex 2 comparatif | Ex 3 non-conforme |
|---|---|---|---|---|
| Huile essentielle d'origan vert | HE origan vert bio SICA (ELIXENC)° | 1 | 1 | 1 |
| Xanthane | Rhodicare CFT (Solvay) | 0 | 0,50 | 0,50 |
| Acrylates/C 10-30 Alkyl Acrylates crosspolymer | PEMULEN TR-1 Polymer (Lubrizol) | 1,40 | 0 | 0 |
| Aminomethyl propanol | AMP Ultra PC 1000, neutralizing Amine (Angus-Dow Chemicals) | 0,8 | 0 | 0 |
| Squalane | Olive Squalane (Deoleo) | 10 | 10 | 10 |
| Acide spiculisporique | Iwata Chemicals | 0 | 0 | 5 |
| Coco-Glucoside | Plantacare 818 UP (BASF) | 0 | 5 | 0 |
| Arginine | Ajinomoto | 0 | 0 | 3 |
| eau | Qsp 100 | Qsp 100 | Qsp 100 | Qsp 100 |

### Préparation des films et méthode expérimentale d'évaluation

La méthode expérimentale d'évaluation est un test d'évaporation en film mince.

Le protocole est le suivant : l'émulsion est appliquée sur un filtre hydrophobe à l'aide d'un tire-film qui se présente sous la forme d'un bras mécanique qui balaie une surface plane à une vitesse contrôlée pour former un film d'épaisseur contrôlée de 50 microns. Le film est ensuite laissé évaporer sous flux d'air contrôlé (Sorbonne). Des prélèvements à différents temps ont été réalisés pour doser la quantité d'huile essentielle restante sur le film par un procédé d'extraction à l'aide de solvants et d'une détection par spectroscopie UV Vis. En raison de la complexité de la composition de l'huile essentielle d'origan vert, seul le composant majoritaire carvarcol (environ 50%) a été dosé.

| | Proportion d'huile essentielle d'origan vert restant dans le film (%) | |
|---|---|---|
| | Moyenne de deux essais N=2 | |
| | t=10 min | t=3h |
| Composition Ex1 (comparatif) | 59,82% | 23,04% |
| Composition Ex2 (comparatif) | 74,84% | 19,24% |
| Composition Ex3 (non-conforme) | 85,38% | 43,01% |

Apres 3 heures de séchage la quantité restante d'huile essentielle d'origan vert est 2 fois plus importante lorsque le film formé a été préparé à partir d'acide spiculisporique.

Les résultats ont montré que l'acide spiculisporique augmente d'un facteur 2 la quantité restante d'huile essentielle d'origan vert après un temps de séchage de 3 heures comparé au coco-glucoside (tensioactif non ionique) ou au polymère réticulé Acrylates/C10-30 Alkyl Acrylates « Pemulen TR1 ».

## Revendications

1. Composition en particulier cosmétique, sous la forme d'une émulsion huile dans l'eau comprenant au moins 1% en poids d'une phase huileuse par rapport au poids total de la composition, et une phase aqueuse **caractérisée en ce qu'**elle comprend :
de 0,1 à 15 %, de préférence de 0,5 à 10 % et de manière encore plus préférée de 1 à 8 % en poids d'acide spiculisporique par rapport au poids total de la composition,
au moins une base choisie parmi les acides aminés et les alcanolamines en une quantité apte à former au moins le monosel de l'acide spiculisporique, ladite base étant en particulier présente en une quantité allant de 0,1 à 20 %, de préférence de 0,5 à 15 et de manière encore plus préférée de 0,6 à 10 % en poids par rapport au poids total de la composition,
moins de 3 %, de préférence moins de 1 % en poids de glycol(s) et/ou de polyol(s) par rapport au poids total de la composition, et étant avantageusement dénuée de glycols et/ou de polyol(s),
au moins un filtre UV et optionellement un additif choisi parmi les matières volatiles odorantes telles que les huiles essentielles et les substances parfumantes, les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

2. Composition selon la revendication précédente dans laquelle la base est un acide aminé choisi parmi l'arginine, la lysine, l'omithine, la citruline et l'histidine, de préférence l'arginine.

3. Composition selon la revendication précédente dans laquelle la base est une alcanolamine choisie parmi les mono-, di-, triéthanolamine, l'isopropanolamine, le 2-amino-2-méthyl-1- propoanol, de préférence la triéthanolamine.

4. Composition selon l'une des revendications précédentes dans laquelle la quantité de base est choisie de façon à former le monosel de l'acide spiculisporique.

5. Composition selon l'une des revendications précédentes dans laquelle la quantité de phase grasse est présente en une quantité d'au moins 5% en poids, de préférence en une quantité allant de 5 à 70 % en poids, plus préférablement de 5 à 60 % en poids par rapport au poids total de la composition.

6. Composition selon l'une des revendications précédentes **caractérisée en ce qu'**elle comprend au moins un agent gélifiant hydrophile en tant qu'épaississant, le gélifiant hydrophile étant en particulier de la gomme de xanthane.

7. Composition selon l'une des revendications précédentes **caractérisée en ce qu'**elle comprend de 0,0001 % à 30 % en poids d'au moins une matière volatile odorante choisie parmi les huiles essentielles et les substances parfumantes par rapport au poids total de la composition.

8. Composition selon la revendication précédente dans laquelle ledit filtre UV est lipophile.

9. Utilisation de la composition selon l'une des revendications précédentes en tant que produit de maquillage, de soin, d'hygiène et/ou de nettoyage des matières kératiniques notament de la peau ou des cheveux.

10. Utilisation non-thérapeutique de la composition selon l'une des revendications 1 à 8 pour protéger les matières kératiniques vis-à-vis des composés polluants atmosphériques notamment choisis parmi le noir de carbone, les oxydes de carbone, les oxydes d'azote, les oxydes de soufre, les composés hydrocarbonés, les composés organiques volatiles, les métaux lourds, les particules fines PM2.5, PM10 et leurs mélanges, et de préférence pour protéger les matières kératiniques des désordres dus aux composés polluants atmosphériques, lesdits désordres étant choisis parmi les peaux grasses, la déshydratation de la peau, l'altération de la désquamation, la diminution du squalène, la diminution de la vitamine E, la pigmentaion, l'augmentation de l'acide lactique.

11. Procédé cosmétique non thérapeutique, de maquillage, de soin, d'hygiène et/ou de nettoyage comprenant une étape d'application de la composition selon l'une des revendications 1 à 8 sur lesdites matières kératiniques.

12. Procédé cosmétique non thérapeutique de protection des matières kératiniques vis-à-vis des composés polluants atmosphériques notamment choisis parmi le noir de carbone, les oxydes de carbone, les oxydes d'azote, les oxydes de soufre, les composés hydrocarbonés, les composés organiques volatiles, les métaux lourds, les particules fines PM2.5, PM10 et leurs mélanges comprenant une étape d'application de la composition selon l'une des revendications 1 à 8 sur lesdites matières kératiniques, le procédé étant en particulier pour protéger les matières kératiniques des désordres dus aux composés polluants atmosphériques, lesdits désordres étant choisis parmi les peaux grasses, la déshydratation de la peau, l'altération de la désquamation, la diminution du squalène, la diminution de la vitamine E, la pigmentaion, l'augmentation de l'acide lactique.

13. Utilisation de l'acide spiculisporique sous forme partiellement ou totalement neutralisée par une base choisie parmi les acides aminés et les alcanolamines dans une composition, en particulier cosmétique, comme agent filmogène pour former un film sur la surface des matières kératiniques, notamment sur la peau ou les cheveux.

14. Utilisation selon la revendication 13, dans laquelle le film formé protège les matières kératiniques vis-à-vis des composés polluants atmosphériques.

15. Utilisation selon la revendication 13, dans laquelle le film formé favorise la répartition homogène d'au moins un actif compris dans la composition sur les matières kératiniques, ledit actif étant préférentiellement un filtre UV, de préférence lipophile, et/ou un additif choisi parmi les charges, les matières colorantes telles que les pigments, les nacres ou les particules à reflet métallique, les agents hydratants, les agents anti-rides ou anti-âge, les agents desquamants, les agents antioxydants, les actifs stimulants la synthèse des macromolécules dermiques et/ou épidermiques, les agents dermodécontractants, les agents dépigmentants, les agents déodorants et leurs mélanges.

## Patentansprüche

1. Zusammensetzung, insbesondere kosmetische Zusammensetzung, in Form einer Ölin-Wasser-Emulsion, umfassend mindestens 1 Gewichtsprozent einer Ölphase, bezogen auf das Gesamtgewicht der Zusammensetzung, und eine wässrige Phase, **dadurch gekennzeichnet, dass** sie Folgendes umfasst:
von 0,1 bis 15 %, vorzugsweise von 0,5 bis 10 % und noch bevorzugter von 1 bis 8 Gewichtsprozent Spiculisporsäure, bezogen auf das Gesamtgewicht der Zusammensetzung, mindestens eine Base, ausgewählt aus Aminosäuren und Alkanolaminen, in einer Menge, die geeignet ist, um mindestens das Monosalz der Spiculisporsäure zu bilden, wobei die Base insbesondere in einer Menge von 0,1 bis 20 %, vorzugsweise von 0,5 bis 15 und bevorzugter von 0,6 bis 10 Gewichtsprozent, bezogen auf das Gesamtgewicht der Zusammensetzung, vorhanden ist,
weniger als 3 Gewichtsprozent, vorzugsweise weniger als 1 Gewichtsprozent Glykol(e) und/oder Polyol(e), bezogen auf das Gesamtgewicht der Zusammensetzung und vorteilhafterweise frei von Glykolen und/oder Polyol(en) ist,
mindestens einen UV-Filter und optional ein Additiv, ausgewählt aus flüchtigen Duftstoffen, wie beispielsweise ätherischen Ölen und Duftstoffen, Füllstoffen, färbenden Stoffen, wie beispielsweise Pigmenten, Perlmutt oder metallisch schimmernden Partikeln, feuchtigkeitsspendenden Wirkstoffen, Anti-Falten- oder Anti-Aging-Wirkstoffen, Schälmitteln, Antioxidantien, Wirkstoffen, die die Synthese dermaler und/oder epidermaler Makromoleküle stimulieren, dermodestreckenden Wirkstoffen, entpigmentierenden Wirkstoffen, Deodorant-Wirkstoffen und deren Gemischen,

2. Zusammensetzung nach dem vorherigen Anspruch, wobei die Base eine Aminosäure ist, ausgewählt aus Arginin, Lysin, Ornithin, Citrulin und Histidin, vorzugsweise Arginin.

3. Zusammensetzung nach dem vorherigen Anspruch, wobei die Base ein Alkanolamin ist, ausgewählt aus Mono-, Di-, Triethanolamin, Isopropanolamin, 2-Amino-2-methyl-1-propoanol, vorzugsweise Triethanolamin,

4. Zusammensetzung nach einem der vorherigen Ansprüche, wobei die Menge an Base auf eine Weise gewählt ist, um das Monosalz der Spiculisporsäure zu bilden.

5. Zusammensetzung nach einem der vorherigen Ansprüche, wobei die Menge an Fettphase in einer Menge von mindestens 5 Gewichtsprozent, vorzugsweise in einer Menge von 5 bis 70 Gewichtsprozent, bevorzugter 5 bis 60 Gewichtsprozent, bezogen auf das Gesamtgewicht der Zusammensetzung, vorhanden ist.

6. Zusammensetzung nach einem der vorherigen Ansprüche, **dadurch gekennzeichnet, dass** sie mindestens ein hydrophiles Geliermittel als Verdickungsmittel umfasst, wobei das hydrophile Geliermittel insbesondere Xanthangummi ist.

7. Zusammensetzung nach einem der vorherigen Ansprüche, **dadurch gekennzeichnet, dass** sie 0,0001 Gewichtsprozent bis 30 Gewichtsprozent mindestens eines flüchtigen Duftstoffs, ausgewählt aus ätherischen Ölen und Duftstoffen, bezogen auf das Gesamtgewicht der Zusammensetzung enthält.

8. Zusammensetzung nach dem vorherigen Anspruch, wobei der UV-Filter lipophil ist.

9. Verwendung der Zusammensetzung nach einem der vorherigen Ansprüche als Schmink-, Pflege-, Hygiene- und/oder Reinigungsprodukt für keratinische Materialien, insbesondere für Haut oder Haare.

10. Nicht-therapeutische Verwendung der Zusammensetzung nach einem der Ansprüche 1 bis 8 zum Schutz von keratinischen Materialien vor atmosphärischen Schadstoffverbindungen, insbesondere ausgewählt aus Ruß, Kohlenstoffoxiden, Stickoxiden, Schwefeloxiden, Kohlenwasserstoffverbindungen, flüchtigen organischen Verbindungen, Schwermetallen, Feinpartikeln PM2.5, PM10 und deren Gemischen, und vorzugsweise zum Schutz von keratinischen Materialien vor Störungen aufgrund von Luftschadstoffverbindungen, wobei die Störungen ausgewählt sind aus fettiger Haut, Austrocknung der Haut, Veränderung der Schuppenbildung, Abnahme von Squalen, Verringerung von Vitamin E, Pigmentierung, Zunahme von Milchsäure.

11. Kosmetisches, nicht therapeutisches Verfahren zum Schminken, zur Pflege, Hygiene und/oder Reinigung, umfassend einen Schritt eines Auftragens der Zusammensetzung nach einem der Ansprüche 1 bis 8 auf die keratinischen Materialien.

12. Kosmetisches, nicht therapeutisches Verfahren zum Schutz von keratinischen Materialien gegenüber atmosphärischen Schadstoffverbindungen, insbesondere ausgewählt aus Ruß, Kohlenoxiden, Stickstoffoxiden, Schwefeloxiden, Kohlenwasserstoffverbindungen, flüchtigen organischen Verbindungen, Schwermetallen, Feinpartikeln PM2.5, PM10 und deren Gemischen, umfassend einen Schritt eines Auftragens der Zusammensetzung nach einem der Ansprüche 1 bis 8 auf die keratinischen Materialien, wobei das Verfahren insbesondere dazu dient, die keratinischen Materialien vor Störungen aufgrund von Luftschadstoffverbindungen zu schützen, wobei die Störungen ausgewählt sind aus fettiger Haut, Austrocknung der Haut, Veränderung der Schuppenbildung, Verringerung von Squalen, Verringerung von Vitamin E, Pigmentierung, Erhöhung von Milchsäure.

13. Verwendung von Spiculisporsäure in teilweise oder vollständig durch eine Base neutralisierter Form, ausgewählt aus Aminosäuren und Alkanolaminen, in einer Zusammensetzung, insbesondere einer kosmetischen Zusammensetzung, als filmbildendes Mittel, um einen Film auf der Oberfläche von keratinischen Materialien, insbesondere auf der Haut oder den Haaren, zu bilden.

14. Verwendung nach Anspruch 13, wobei der gebildete Film die keratinischen Materialien vor Luftschadstoffverbindungen schützt.

15. Verwendung nach Anspruch 13, wobei der gebildete Film die homogene Verteilung mindestens eines in der Zusammensetzung enthaltenen Wirkstoffs auf den keratinischen Materialien begünstigt, wobei der Wirkstoff vorzugsweise ein UV-Filter, vorzugsweise ein lipophiler Filter und/oder ein Additiv ist, ausgewählt aus Füllstoffen, Farbmaterialien, wie beispielsweise Pigmenten, Perlmutt oder metallisch schimmernde Partikel, Feuchtigkeitsspendern, Anti-Falten- oder Anti-Aging-Wirkstoffen, Schälmitteln, Antioxidationsmitteln, Wirkstoffen, die die Synthese dermaler und/oder epidermaler Makromoleküle stimulieren, Dermodontraktionsmitteln, Depigmentierungsmitteln, Deodorant-Mitteln und deren Gemischen.

## Claims

1. A composition, in particular cosmetic composition, in the form of an oil-in-water emulsion comprising at least 1 % by weight of an oily phase relative to the total weight of the composition, and an aqueous phase **characterized in that** it comprises:
from 0.1 % to 15 %, preferably from 0.5 % to 10 % and even more preferably from 1 % to 8 % by weight of spiculisporic acid relative to the total weight of the composition,
at least one base chosen from amino acids and alkanolamines in an amount capable of forming at least the monosalt of spiculisporic acid, said base being comprised in particular in an amount ranging from 0.1 to 20 %, preferably from 0.5 to 15 % and further preferably from 0.6 to 10 % by weight relative to the total weight of the composition,
less than 3%, preferably less than 1% by weight of glycol(s) and/or polyol(s) relative to the total weight of the composition, and advantageously being free of glycols and/or polyol(s),
at least one UV-screening agent and optionally an additive chosen from odorous volatile materials such as essential oils and fragrancing substances, fillers, dyestuffs such as pigments, nacres or particles with a metallic tint, moisturizers, antiwrinkle or antiaging agents, desquamating agents, antioxidants, active agents for stimulating the synthesis of dermal and/or epidermal macromolecules, dermo-relaxant agents, depigmenting agents and deodorants, and mixtures thereof.

2. The composition as claimed in the preceding claim, wherein the base is an amino acid chosen from arginine, lysine, ornithine, citrulline and histidine, preferably arginine.

3. The composition as claimed in the preceding claim, wherein the base is an alkanolamine chosen from mono-, di- and triethanolamine, isopropanolamine and 2-amino-2-methyl-1-propanol, preferably triethanolamine.

4. The composition as claimed in one of the preceding claims, wherein the amount of base is chosen so as to form the monosalt of spiculisporic acid.

5. The composition as claimed in one of the preceding claims, wherein the amount of fatty phase is present in an amount of at least 5 % by weight, preferably in an amount ranging from 5 % to 70 %, more preferably from 5 % to 60 % by weight relative to the total weight of the composition.

6. The composition as claimed in one of the preceding claims, **characterized in that** it comprises at least one hydrophilic gelling agent as thickener, the hydrophilic gelling agent particularly being xanthan gum.

7. The composition as claimed in one of the preceding claims, **characterized in that** it comprises from 0.0001 % to 30 % by weight of at least one odorous volatile material chosen from essential oils and fragrancing substances, relative to the total weight of the composition.

8. The composition as claimed in the preceding claim, wherein said UV-screening agent is lipophilic.

9. Use of the composition as claimed in one of the preceding claims, as a makeup, care, hygiene and/or cleansing product for keratin materials, especially the skin or the hair.

10. Non-therapeutic use of the composition as claimed in one of claims 1 to 8, for protecting keratin materials against atmospheric pollutant compounds chosen especially from carbon black, carbon oxides, nitrogen oxides, sulfur oxides, hydrocarbon-based compounds, volatile organic compounds, heavy metals, PM2.5 and PM10 fine particles, and mixtures thereof, and preferably for protecting keratin materials against disorders due to atmospheric pollutant compounds, said disorders being selected from among oily skin, skin dehydration, desquamation impairment, a decrease in squalene, a decrease in vitamin E, pigmentation, an increase in lactic acid.

11. A nontherapeutic cosmetic makeup, care, hygiene and/or cleansing process comprising a step of applying the composition as claimed in one of claims 1 to 8 to said keratin materials.

12. A nontherapeutic cosmetic process for protecting keratin materials against atmospheric pollutant compounds chosen especially from carbon black, carbon oxides, nitrogen oxides, sulfur oxides, hydrocarbon-based compounds, volatile organic compounds, heavy metals, PM2.5 and PM10 fine particles, and mixtures thereof, comprising a step of applying the composition as claimed in one of claims 1 to 8 to said keratin materials, the process being in particular intended to protect keratin materials against disorders due to atmospheric pollutant compounds, said disorders being selected from among oily skin, skin dehydration, desquamation impairment, a decrease in squalene, a decrease in vitamin E, pigmentation, an increase in lactic acid.

13. Use of spiculisporic acid in a form partially or totally neutralized with a base chosen from amino acids and alkanolamines in a composition, in particular a cosmetic composition, as a film-forming agent for forming a film on the surface of keratin materials, especially on the skin or the hair.

14. The use as claimed in claim 13, wherein the film formed protects keratin materials against atmospheric pollutant compounds.

15. The use as claimed in claim 13, wherein the film formed promotes the homogeneous distribution of at least one active agent included in the composition on keratin materials, said active agent preferentially being a UV-screening agent, which is preferably lipophilic, and/or an additive chosen from fillers, dyestuffs such as pigments, nacres or particles with a metallic tint, moisturizers, antiwrinkle or antiaging agents, desquamating agents, antioxidants, active agents for stimulating the synthesis of dermal and/or epidermal macromolecules, dermo-relaxant agents, depigmenting agents and deodorants, and mixtures thereof.
